# EUROPÄISCHE PATENTSCHRIFT

(11) **EP 2 203 116 B1**
(45) Veröffentlichungstag und Bekanntmachung des Hinweises auf die Patenterteilung: **05.03.2014**
(21) Anmeldenummer: 08785265.3
(22) Anmeldetag: 31.07.2008
(51) Int. Cl.: A61B 5/022

(54) **VORRICHTUNG UND VERFAHREN ZUM BESTIMMEN DES KNÖCHEL-ARM-INDEXES EINES PROBANDEN**
DEVICE AND METHOD FOR DETERMINING THE ANKLE-ARM INDEX OF A TEST PERSON
DISPOSITIF ET PROCÉDÉ DE DÉTERMINATION DE L'INDEX CHEVILLE/BRAS D'UN SUJET

(30) Priorität: 31.10.2007 DE 102007052222
(43) Veröffentlichungstag der Anmeldung: 07.07.2010
(73) Patentinhaber: Bosch + Sohn GMBH & CO. KG, 72417 Jungingen (DE)
(72) Erfinder: WELTE, Wolfgang, 72351 Geislingen (DE)
(74) Vertreter: Müller - Hoffmann & Partner
(86) Internationale Anmeldenummer: PCT/EP2008/006324
(87) Internationale Veröffentlichungsnummer: WO 2009/056182

(56) Entgegenhaltungen:
- EP-A- 1 319 363
- EP-A- 1 393 670
- EP-A- 1 679 031
- US-A1- 2002 133 082
- US-A1- 2006 258 944
- PAN CHENG-RUI ET AL: "Comparison of three measures of the ankle-brachial blood pressure index in a general population." HYPERTENSION RESEARCH : OFFICIAL JOURNAL OF THE JAPANESE SOCIETY OF HYPERTENSION JUN 2007, Bd. 30, Nr. 6, Juni 2007 (2007-06), Seiten 555-561, XP002503400 ISSN: 0916-9636
- GREENLAND P ET AL: "Prevention Conference V: Beyond secondary prevention: identifying the high-risk patient for primary prevention: noninvasive tests of atherosclerotic burden: Writing Group III." CIRCULATION 4 JAN 2000, Bd. 101, Nr. 1, 4. Januar 2000 (2000-01-04), Seiten E16-E22, XP002503401 ISSN: 1524-4539

## Beschreibung

Die Erfindung betrifft eine Vorrichtung zum Bestimmen eines Knöchel-Arm-Indexes eines Probanden mittels einer Zentraleinheit zum oszillometrischen Messen von Blutdrücken an den Extremitäten des Probanden.

Der Knöchel-Arm-Index ist ein Indexwert zum Beurteilen des kardiovaskulären Zustandes eines Patienten, insbesondere zum Beurteilen eines Vorliegens einer arteriellen Verschlusskrankheit (AVK) bzw. einer peripheren arteriellen Verschlusskrankheit (pAVK). Einer AVK liegt häufig eine Arteriosklerose zugrunde, sie kann aber auch auf andere Störungen der arteriellen Durchblutung an den Extremitäten durch eine Einengung von Blutgefäßen zurück gehen, wie etwa Entzündungen. Engstellungen (sog. Stenosen) oder Verschlüsse (sog. Trombosen oder Embolien). Derartige Verengungen von Blutgefäßen führen in der Regel zu einer hämodynamischen Kompensation, also zu einem niedrigeren Blutdruck in der betroffenen Extremität. Unterschiedliche Blutdrücke in den Extremitäten lassen somit auf eine hämodynamische Kompensation und damit auf das Vorliegen einer AVK schließen. Je nach Höhe der Druckunterschiede können Rückschlüsse auf verschiedene Stadien bzw. Schweregrade einer derartigen Erkrankung gezogen werden.

Zur einheitlichen Beurteilung und Kategorisierung derartiger Unterschiede des Blutdruckes der Extremitäten eines Probanden, kann für eine Körperhälfte des Probanden jeweils ein Knöchel-Arm-Index ermittelt werden. Es existiert also ein Knöchel-Arm-Index links und ein Knöchel-Arm-Index rechts. Diese Indexwerte werden auch als ABI-Werte bezeichnet (aus engl. Ankle-Brachial-Index, Knöchel-Arm-Index).

Der Knöchel-Arm-Index stellt das Verhältnis der Blutdrücke von oberen und unteren Extremitäten, insbesondere an Oberarmen und Knöcheln dar, wobei der Index auf verschiedene Weisen ermittelt werden kann.

Üblicherweise wird der Knöchel-Arm-Index aus dem Quotienten des Druckwerts der entsprechenden unteren Extremität und der halben Summe der jeweiligen Druckwerte der oberen Extremitäten, ermittelt. Somit ergibt sich beispielsweise der Knöchel-Arm-Index links aus dem Quotienten des Druckwerts des linken Beins, welcher beispielsweise am linken Knöchel bestimmt wurde, und der halben Summe der Druckwerte der Arme, welche beispielsweise an den jeweiligen Oberarmen bestimmt wurden, die somit den Mittelwert der Druckwerte der Beine darstellt.

Ein Quotient liegt bei gesunden Menschen regelmäßig zwischen 0,9 und 1,2, während Quotienten von unter 0,9 bereits auf den Beginn einer arteriellen Verschlusskrankheit wie einer Arteriosklerose schließen lassen. Je niedriger der Indexwert ist, desto schwerwiegender ist in der Regel die zugrunde liegende arterielle Verschlusskrankheit. Erhöhte ABI-Werte von über 1,2 können Indikatoren für eine Gefäßverkalkung wie beispielsweise einer Mediasklerose sein, welche nicht unter die Kategorie AVK fällt. Somit sind auch weitere Erkrankungen mithilfe des Knöchel-Arm-Indexes diagnostizierbar.

Aus EP 1 319 363 A1 ist ein Diagnosegerät für Arteriosklerose bekannt, welches vier aufblasbare Manschetten für Extremitäten eines Probanden aufweist, die jeweils mit einer Blutdruckmesskonsole über Röhrchen verbunden sind. Eine Einrichtung zum Bestimmen der Blutdrücke der oberen Extremitäten ist neben einer Einrichtung zum Bestimmen der Blutdrücke der unteren Extremitäten bereitgestellt und die Ausgänge dieser beiden Einrichtungen werden in eine Einrichtung zum Bestimmen eines Knöchel-Arm-Blutdruck-Indexes eingegeben.

Aus US2006/0258944 A1 sind eine Vorrichtung und ein Verfahren zum Ausgeben einer Bioinformation bekannt, wobei zwei oder mehrere Paare von Blutdruckwerten gesammelt werden. Eine Steuereinheit sammelt Messdaten der oberen Extremitäten, während eine weitere Steuereinheit Daten der unteren Extremitäten bei Messungen mit entsprechenden Manschetten aufweist.

In Pan Cheng-Rui et al.: Comparison of three measures of the ankle-brachial-blood pressure index in a general population" HYPERTENSION RESEARCH: OFFICIAL JOURNAL OF THE JAPANESE SOCIETY OF HYPERTENSION JUN 2007, Bd. 30, Nr. 6, Juni 2007, Seiten 555-561, XP002503400 ISSN: 0916-9636 ist ein Vergleich von drei Messungen des Knöchel-Arm-Blutdruck-Indexes in einer bestimmten Population beschrieben. Es wird eine automatisierte oszillometrische Technik mit einer simultanen Knöchel- und Arm-Messung erwähnt.

Der Erfindung liegt die Aufgabe zugrunde, eine Vorrichtung zum Bestimmen eines Knöchel-Arm-Indexes eines Probanden arizugeben. Außerdem liegt der Erfindung die Aufgabe zugrunde, ein Verfahren zum Bestimmen eines Knöchel-Arm-Indexes eines Probanden anzugeben.

Erfindungsgemäß wird die Aufgabe durch eine Vorrichtung gemäß Patentanspruch 1 gelöst. Ein entsprechendes Verfahren ist in Anspruch 9 definiert. Vorteilhafte Weiterentwicklungen der Erfindung sind in den abhängigen Ansprüchen angegeben.

Eine erfindungsgemäße Vorrichtung dient der Bestimmung eines Knöchel-Arm-Indexes eines Probanden, insbesondere eines menschlichen Patienten, und weist vier Manschetten auf, die an jeweils einer der Extremitäten, also den Armen und Beinen des Probanden anlegbar sind. Insbesondere sind die Manschetten an den Oberarmen bzw. den Knöcheln des Probanden anlegbar und können an den Extremitäten festgeschnallt, angeklettet oder in sonstiger Weise angebracht werden. Ferner können die Manschetten jeweils wenigstens einen mit einem Gas oder einer Flüssigkeit füllbaren Hohlkörper aufweisen, wodurch die Manschetten aufblasbar bzw. aufpumpbar sind, um somit einen Druck auf die jeweilige Extremität auszuüben.

Die Vorrichtung weist zudem Drucksensoren sowie eine Zentraleinheit auf, welche eine oszillometrische Messung von Blutdrücken des Probanden vornehmen kann. Dabei kann typischerweise für jede Extremität ein Blutdruck ermittelt werden, also je ein Blutdruck für den rechten Arm, den linken Arm, das rechte Bein und das linke Bein.

Für unterschiedliche Größen bzw. körperliche Konstitutionen von Probanden können die Manschetten in verschiedenen Größen bzw. Umfängen gefertigt sein, um ihre Anwendung beispielsweise für erwachsene Probanden verschiedener Statur ebenso wie für Kinder zu ermöglichen, damit Knöchel-Arm-Indizes gleichermaßen bestimmbar sind.

Selbstverständlich ist es auch möglich, einen Knöchel-Arm-Index eines Tieres, wie beispielsweise eines Hundes oder einer Katze, mit der Vorrichtung zu bestimmen. Dann sind die Manschetten entsprechend an den Vorder- und Hinterbeinen bzw. den Pfoten anlegbar.

Die Drucksensoren der Vorrichtung können beispielsweise Passivdrucksensoren, Relativdrucksensoren, Absolutdrucksensoren oder Differenzdrucksensoren sein. Dabei können die Drucksensoren elektronische Drucksensoren sein. Möglich ist der Einsatz von piezoresistiven, piezoelektrischen, frequenzanalogen, kapazitiven, induktiven, Hallelement-basierten oder weiteren elektronischen Drucksensoren. Wichtig ist hierbei. dass eine oszillometrische Messung mit den Sensoren durchführbar ist.

Durch die Drucksensoren wird der Blutdruck der entsprechenden Extremität sensorisiert. Diese Drucksensoren sind von der Zentraleinheit auslesbar, wodurch für jede Extremität, an die eine Manschette angelegt ist, der jeweilige Blutdruck bestimmbar ist. Dabei wird ein oszillometrisches bzw. oszillatorisches Messprinzip verwendet. Die pulsatilen Druckänderungen in den Arterien unter den Manschetten erzeugen periodische bzw. oszillierende Druckänderungen an den darüber liegenden Manschetten. Diese Oszillationen an den Manschetten werden an die Drucksensoren weitergegeben und dort als indirektes Maß für pulsatile Druckänderungen in den unter den Manschetten liegenden Arterien zum Messen des Blutdrucks herangezogen. Hierbei können der systolische Druck und der diastolische Druck entweder aus den gemessenen maximalen Amplitudenwerten errechnet, oder systolischer sowie diastolischer Blutdruck jeweils sensorisiert werden.

Eine oszillatorische Messung bedarf üblicherweise nicht der Durchführung durch einen Arzt oder einen sonstigen Spezialisten und kann daher auch von nicht speziell qualifizierten Personen durchgeführt werden. Somit kann eine erfindungs-gemäße Vorrichtung auch von einer Arzthelferin oder einem Arzthelfer eingesetzt werden. Der Einsatz der Vorrichtung kann aber auch vollautomatisch durch eine Maschine bzw. einen Rechner, wie beispielsweise eine automatisierte Patientenüberwachung in einer Klinik, oder an einem Wohnsitz eines Probanden geschehen.

Bei einer Ausführungsform sind die Drucksensoren in der Zentraleinheit angeordnet und so mit den Manschetten verbunden, dass Drücke an den Manschetten durch einen jeweiligen Drucksensor der Zentraleinheit sensorisiert werden können. Die Drucksensoren sind über Gas- oder Flüssigkeitsschläuche mit den Manschetten verbunden, so dass die Wirkungen von Drücken, die an den Manschetten auftreten bis zur Zentraleinheit geleitet werden, wo sie von den Drucksensoren erfasst werden können. Dabei haben die Schläuche die Eigenschaft, dass sie sich nicht durch die auftretenden Drücke bzw. Druckschwankungen deformieren, um die Drucksensorisierung nicht durch Druckänderungen zu verfälschen.

Gemäß einer weiteren Ausführungsform sind die Drucksensoren in den Manschetten angeordnet, so dass Drücke direkt an den Manschetten sensorisiert werden können. Die Drucksensoren sind hier über elektrische Zuleitungen oder dratlosen Übertragungsstrecken zur Datenübertragung sowie zur Leistungsversorgung mit der Zentraleinheit verbunden.

Bei einer Ausführungsform der Vorrichtung ermittelt die Zentraleinheit Druck-werte der jeweiligen Blutdrücke des Probanden durch die Drucksensoren. Der Druck an den Sensoren variiert periodisch entsprechend dem Puls in den Blutgefäßen bzw. des Herzens sowie synchron zu der periodischen also quasi oszillierenden Kontraktion der Blutgefäße, an denen die Sensoren sensorisieren. Die systo-lischen und diastolischen Blutdrücke werden in der Zentraleinheit ausgewertet und die jeweiligen Blutdrücke an den einzelnen Extremitäten werden so ermittelt.

Die Druckwerte sind von der Zentraleinheit gleichzeitig an mehreren Manschetten ermittelbar, so dass für die jeweiligen Extremitäten die Druckwerte gleichzeitig ermittelbar sind. Da Blutdrücke in einer Extremität bei aufeinander folgenden Messungen an den verschiedenen Extremitäten voneinander abweichen können, ist eine Bestimmung von ABI-Werten in einem solchen Fall einer Ungenauigkeit unterworfen. Diese Ungenauigkeit tritt bei einem gleichzeitigen Messen der Blutdrücke in den Extremitäten nicht auf. Somit ist durch die Vorrichtung eine sehr genaue Bestimmung von Knöchel-Arm-Indexwerten möglich, wodurch auch ein Vergleich der Indexwerte sowie eine Diagnose genauer ist. Des Weiteren ist eine Bestimmung von ABI-Werten mit dieser Vorrichtung schneller möglich, als in einem Fall, in welchem Blutdrücke an den Extremitäten nacheinander gemessen werden.

Gemäß einer weiteren Ausführungsform ist durch die Zentraleinheit ein Knöchel-Arm-Index des Probanden aus zumindest zwei ermittelten Druckwerten bestimmbar. Ein Knöchel-Arm-Index rechts kann beispielsweise aus dem Quotienten des Druckwertes des rechten Beines und dem Druckwert eines Armes bestimmt werden.

Vorzugsweise werden jedoch drei Druckwerte für die Bestimmung eines Knöchel-Arm-Indexes bzw. eines ABI-Wertes verwendet. Der Knöchel-Arm-Index links ist aus den Druckwerten des linken Knöchels und der beiden Oberarme durch die Zentraleinheit bestimmbar. Für die rechte Körperhälfte des Probanden ist der Knöchel-Arm-Index rechts aus den Druckwerten des rechten Knöchels und der beiden Oberarme bestimmbar. Hierzu wird jeweils der Quotient aus dem Druckwert des jeweiligen Knöchels und der halben Summe der jeweiligen Druckwerte (also dem Mittelwert der beiden jeweiligen Druckwerte) der Oberarme gebildet. Diese Bestimmung der ABI-Werte wird vorzugsweise dann durchgeführt, wenn die Druckwerte der Oberarme einen Druckunterschied kleiner 10mmHg aufweisen.

Falls der Unterschied der Druckwerte der beiden Oberarme größer als 10mmHg beträgt, wird, wie oben beschrieben, ein ABI-Wert aus einem Druckwert eines Beines und einem Druckwert eines Armes ermittelt. Dabei wird der ABI-Wert links bzw. rechts aus dem Quotienten des Druckwerts des linken bzw. des rechten Knöchels und dem Druckwert des Armes mit dem höheren Druck errechnet.

Da alle Druckwerte der jeweiligen Extremitäten gleichzeitig erfassbar sind, können durch die Zentraleinheit die Knöchel-Arm-Indizes links und rechts gleichzeitig bestimmt werden. Durch einen Vergleich dieser Indizes ist über die Erstellung eines Befundes über das Vorliegen beispielsweise einer AVK möglich, und darüber hinaus auch die Bestimmung, in welcher Weise sich Erkrankungsstadien verschiedener Extremitäten voneinander unterscheiden. Damit lässt sich feststellen, ob ein Proband unter einer AVK aller Extremitäten leidet oder ob lediglich eine Extremität besonders betroffen ist, was sehr anschaulich Rückschlüsse über den Allgemeinzustand des Probanden bietet.

Gemäß einer weiteren Ausführungsform der Vorrichtung ist die Zentraleinheit mit einer Anzeigeeinheit verbunden, welche die gemessenen Druckwerte der Extremitäten einzeln oder die daraus bestimmten Knöchel-Arm-Indizes anzeigen kann. Dies kann vorzugsweise über einen Bildschirm mit der Fähigkeit zur grafischen Darstellung erfolgen. Möglich ist auch die Verwendung von bistabilen Anzeigeelementen oder einfachen Lampen bzw. Licht emittierenden Dioden. Daneben ist auch eine akustische Signalisierung, beispielsweise für das Erreichen von wählbaren Schwellenwerten gemessener Drücke oder Indizes, für eine Darstellung von Druckwerten oder Knöchel-Arm-Indizes möglich.

Gemäß einer weiteren Ausführungsform der Vorrichtung weist die Zentraleinheit wenigstens eine mit aufblasbaren Manschetten über Luftschläuche verbundene Luftpumpe auf, so dass die Zentraleinheit die Manschetten mittels der wenigstens einen Luftpumpe aufblasen kann. Hierzu weisen die Manschetten Luftkammern auf und können durch ihre Inflation Druck auf die Extremitäten ausüben, an welchen sie jeweils angelegt sind. Üblicherweise kommt als Inflationsgas Luft zum Einsatz, es kann aber selbstverständlich auch jedes beliebige Gas bzw. Gasgemisch verwendet werden. In entsprechender Weise können statt Gasen auch Flüssigkeiten zum Füllen der Manschetten verwendet werden. Dann ist eine Luftpumpe eine Pumpe zum Pumpen von Flüssigkeiten und die Luftschläuche sind Schläuche zum Führen von Flüssigkeiten.

Möglich ist die Verwendung von vier Luftpumpen in der Zentraleinheit, so dass jede Manschette über eine separate Luftpumpe aufblasbar ist. In diesem Fall bilden jeweils eine Pumpe und eine Manschette ein separates Drucksystem. Dadurch kann jede Manschette auf ein eigenes Druckniveau aufgeblasen werden. Außerdem beeinflusst der Druck, der von einer Manschette an die Zentraleinheit übertragen wird nicht den Druck, der von den übrigen Manschetten übertragen wird. Auf diese Weise ist eine sehr genaue Messung der Blutdrücke und folglich eine sehr genaue Bestimmung der ABI-Werte möglich.

Beim Aufblasen der Manschetten können bei dieser Ausführungsform bereits die Blutdrücke der Extremitäten gemessen werden, um festzustellen, auf welches Druckniveau die jeweiligen Manschetten für eine optimale Druckwirkung auf die jeweilige Extremität aufgepumpt werden sollen. Dadurch sind alle Manschetten auf das benötigte Druckniveau aufblasbar.

Es ist jedoch auch möglich, nur eine Pumpe zum Befüllen aller Manschetten bereitzustellen. Dazu weist die Zentraleinheit lediglich eine Pumpe auf, sowie je ein Rückschlagventil für jeden Schlauch von der Pumpe zu jeweils einer Manschette. Durch diese Rückschlagventile wird verhindert, dass der Druck, der von einer Manschette an die Zentraleinheit bzw. den entsprechenden Drucksensor über eine Zuführung übertragen wird, von Drücken anderer Manschetten beeinflusst wird. Es liegen somit faktisch ebenfalls vier separate Drucksysteme vor, so dass mit nur einer Luftpumpe dennoch sehr genaue Druckmessungen und folglich sehr genaue Bestimmungen der Knöchel-Arm-Indizes möglich sind.

Beim Aufblasen der Manschetten können auch bei dieser Version bereits die Blutdrücke der Extremitäten gemessen werden, um festzustellen, auf welches Druckniveau die jeweiligen Manschetten für eine optimale Druckwirkung auf die jeweilige Extremität aufgepumpt werden sollen. Dadurch sind alle Manschetten auf das benötigte Druckniveau aufblasbar.

Es wird jedoch für alle Manschetten einheitlich das höchste Druckniveau verwendet.

Durch das einheitliche Aufpumpen aller Manschetten auf das gleiche Druckniveau wird die Gleichzeitigkeit der Messung weiter verbessert.

Eine Manschette der Vorrichtung weist gemäß einer Ausführungsform ein Ventil zum Regeln des Auslassens von durch die Luftpumpe zugeführter Luft auf. Da die Zentraleinheit jedoch über Luftschläuche mit den Manschetten verbunden ist, kann auch die Zentraleinheit wenigstens ein Ventil zum Regeln des Auslassens von durch die Luftpumpe zugeführter Luft aus wenigstens einer der Manschetten aufweisen. Wichtig ist dabei, dass Luft aus jeder Manschette separat auslassbar ist. Mit größerem Luftvolumen aufgepumpte Manschetten können demnach bei Bedarf mit stärkerem Luftstrom entleert werden, während schwächer aufgepumpte Manschetten mit kleinerem Luftstrom entleert werden können, so dass die Drücke in den Manschetten synchronisiert immer auf dem gleichen Niveau sind. Dadurch wird erreicht, dass die Entleerungszeiten der Manschetten, welche den Messzeiträumen für die Druckmessungen entsprechen, annähernd gleich lang sind. Dies hat zur Folge, dass die Messungen der Blutdrücke und damit der ABI-Werte selbst bei unterschiedlich stark aufgeblasenen Manschetten gleichzeitig erfolgen können. Entsprechend der obigen Beschreibung gilt selbstverständlich, dass beim Einsatz von Flüssigkeiten die Ventile zum Regeln eines Flüssigkeitsstroms geeignet sind.

Durch den Druck, welchen die Manschetten durch ihre Befüllung auf die Extremitäten ausüben, wird der Blutstrom in der jeweiligen Extremität behindert. Damit das Blut wieder fließen kann, wird der Druck in den Manschetten sukzessive reduziert. Allerdings ist entscheidend, dass ein Druck auf die Extremität ausgeübt wird, der veränderbar ist. Aus diesem Grund ist es nicht zwingend notwendig, aufblasbare Manschetten für die Druckerzeugung zu verwenden. Beispielsweise kann Druck auch von außen auf die Manschetten ausgeübt werden, indem beispielsweise eine Person einen Finger mit veränderlicher Kraft daran andrückt oder etwa durch festziehbare und wieder lösbare Schlingen oder Schlaufen.

Zum Steuern bzw. Regeln der Ventile weist die Zentraleinheit gemäß einer Ausführungsform der Vorrichtung eine Ventilsteuerung auf. Dabei kann die Ventilsteuerung das Auslassen von Luft gemäß einer Voreinstellung steuern, so dass ein gleichmäßiges Auslassen von Luft möglich ist. Dies geschieht beispielsweise durch Abspielen eines entsprechenden Steuerprogramms zum Steuern der Ventile. Mehrere Voreinstellungen können eine Anpassbarkeit des Auslassens von Luft aus Manschetten unterschiedlicher Größe, beispielsweise in großen Größen für Kinder oder in kleinen Größen für Erwachsene bieten. Die Charakteristik des Auslassens von Luft kann für die Ventile aber auch durch einen Bediener verändert werden, um beispielsweise verschiedene Manschetten unterschiedlich schnell zu entleeren, um somit etwa eine Bestimmung von Knöchel-Arm-Indizes bei Probanden mit Normanomalien der Extremitäten zu erlauben. Dazu weist die Zentraleinheit bzw. die Ventilsteuerung ein Eingabegerät auf, worüber ein Benutzer der Vorrichtung das Öffnen bzw. Schließen der Ventile steuern kann.

Ein Regeln der Pumpe bzw. Pumpen durch eine Pumpensteuerung oder durch eine kombinierte Pumpen-Ventilsteuerung kann dadurch erreicht werden, dass während des Aufpumpens der Manschetten bereits Blutdrücke der jeweiligen Extremitäten gemessen werden. Entsprechend den Druckniveaus der Manschetten regelt die Pumpensteuerung die Pumpe bzw. Pumpen, so dass jede Manschette auf ihr optimales Druckniveau aufgeblasen wird.

Ein Verfahren zum Bestimmen eines Knöchel-Arm-Indexes eines Probanden. umfasst die folgenden Schritte:
- Anlegen je einer mit einer Drucksensoren aufweisenden Zent-raleinheit verbundenen Manschette an den Extremitäten des Probanden;
- Entleeren von mit einem großen Volumen aufgepumpten Manschetten mit einem stärkeren Luftstrom und Entleeren von schwächer aufgepumpten Manschetten mit einem kleineren Luftstrom, bei einem
- Einhalten eines gleichen Druckniveaus in den Manschetten, und einem
- gleichmäßigen Auslassen von Luft aus den Manschetten,
- gleichzeitiges oszillometrisches Messen von Blutdrücken an den jeweiligen Extremitäten zum Ermitteln je eines Druckwertes der Blutdrücke, wobei durch die Manschetten Drücke mit einem einheitlichen Druckniveau auf die entsprechenden Extremitäten ausgeübt werden und die Drücke derart verringert werden, dass das entsprechend verringerte Druckniveau einheitlich bleibt; und
- Bestimmen eines Knöchel-Arm-Indexes aus den Druckwerten.

Das Anlegen der Manschetten an den Extremitäten kann durch das Anlegen einer Manschette an einem Oberarm des Probanden und das Anlegen einer Manschette an einem Knöchel des Probanden geschehen. Es können auch Manschetten an beiden Oberarmen sowie an beiden Knöcheln des Probanden angelegt werden.

Das Bestimmen eines Knöchel-Arm-Indexes links erfolgt nach einem Verfahren durch Errechnen des Quotienten des Druckwerts des entsprechenden linken Knöchels und der halben Summe der jeweiligen Druckwerte der Oberarme. Ein Knöchel-Arm-Index rechts wird durch Errechnen des Quotienten des Druckwerts des entsprechenden rechten Knöchels und der halben Summe der jeweiligen Druckwerte der Oberarme.

Gemäß einem weiteren Verfahren erfolgt das Bestimmen eines Knöchel-Arm-Indexes links durch Errechnen des Quotienten des Druckwerts des entsprechenden linken Knöchels und dem höheren der jeweiligen Druckwerte der beiden Ober-arme. Ein Knöchel-Arm-Index rechts erfolgt durch Errechnen des Quotienten des Druckwerts des entsprechenden rechten Knöchels und dem höheren der beiden jeweiligen Druckwerte der Oberarme. Erfindungsgemäß können zwei oder mehrere Vorrichtungen zum oszillometrischen Messen von Blutdruck zum Bestimmen eines Knöchel-Arm-Indexes einer Extremität eines Probanden verwendet werden.

Diese und weitere Vorteile und Merkmale der Erfindung werden nachfolgend anhand von Beispielen unter Zuhilfenahme der begleitenden Figuren näher erläutert. Es zeigen:
**Figur 1** eine in skizzierter Form dargestellte Vorrichtung gemäß einer ersten Ausführungsform der Erfindung;
**Figur 2** eine in skizzierter Form dargestellte Vorrichtung gemäß einer weiteren Ausführungsform der Erfindung;
**Figur 3** eine in skizzierter Form dargestellte Vorrichtung gemäß einer weiteren Ausführungsform der Erfindung; und
**Figur 4** eine in skizzierter Form dargestellte Vorrichtung gemäß einer weiteren Ausführungsform der Erfindung.

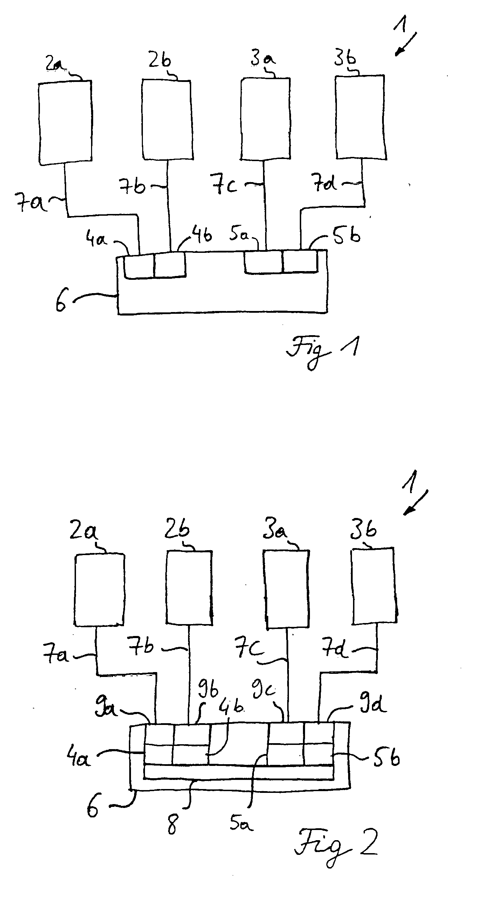

Figur 1 zeigt eine Skizze einer Vorrichtung 1 zum Bestimmen eines Knöchel-Arm-Indexes eines Probanden mit vier Manschetten 2a, 2b, 3a, 3b, welche mit jeweils einem Drucksensor 4a, 4b, 5a, 5b verbunden sind. Die Drucksensoren sind in einer Zentraleinheit 6 angeordnet und die Manschetten 2a, 2b, 3a, 3b sind mit der Zentraleinheit 6 über Zuführungen 7a, 7b, 7c, 7d verbunden. Wie dargestellt, sind bei dieser Ausführungsform genauer gesagt die Drucksensoren 4a, 4b, 5a, 5c der Zentraleinheit 6 mit den Manschetten 2a, 2b, 3a, 3b verbunden.

Die Zuführungen 7a, 7b, 7c, 7d können elektrische Leitungen zum Übertragen eines elektrischen bzw. elektronischen Signals oder für eine elektrische Stomversorgung sein, für den Fall, dass in den Manschetten 2a, 2b, 3a, 3b beispielsweise weitere Sensoren anordenbar sein sollen. Die Zuführungen 7a, 7b, 7c, 7d können dabei aber auch stellvertretend für drahtlose Kommunikationsstrecken stehen. Bei dieser Ausführungsform übernehmen die Zuführugnen 7a, 7b, 7c, 7d jedoch hauptsächlich die Aufgabe, an den Manschetten durch deren Anlegen an die Extremitäten des Probanden auftretende Drücke an die Drucksensoren 4a, 4b, 5a, 5b der Zentraleinheit 6 zu übertragen.

Die Manschetten 2a, 2b, 3a, 3b sind an jeweils eine der Extremitäten eines Probanden anlegbar. Insbesondere sind zwei Manschetten 2a, 2b, die jeweils mit einem Drucksensor 4a, 4b verbunden sind, an den Oberarmen anlegbar, während die anderen beiden Manschetten 3a, 3b, die mit jeweils einem Drucksensor 5a, 5b verbunden sind, an den Knöcheln des Probanden anlegbar sind. Die Zentraleinheit 6 kann durch die Drucksensoren 4a, 4b, 4c, 4d oszillometrisch Blutdrücke des Probanden messen, insbesondere jeweils einen Blutdruck für jede Extremität.

Figur 2 zeigt eine Skizze einer Vorrichtung 1 zum Bestimmen eines Knöchel-Arm-Indexes eines Probanden. Einzelheiten zu Figur 2 werden nur in soweit erläutert, wie sie Unterschiede zu der Ausführungsform nach Figur 1 betreffen.

Die Zentraleinheit 6 der Vorrichtung 1 weist eine Ventilsteuerung 8 sowie vier Luftpumpen 9a, 9b, 9c, 9d auf. Mit den Luftpumpen 9a, 9b, 9c, 9d ist Luft über die Zuführungen 7a, 7b, 7c, 7d in die Manschetten 2a, 2b, 3a, 3b einbringbar. Hierbei sind die Zuführungen 7a, 7b, 7c, 7d geeignet, Luft zu führen und weisen zum Beispiel Schläuche auf. Insbesondere sind die Zuführungen 7a, 7b, 7c, 7d geeignet, Drücke, die an den Manschetten 2a, 2b, 3a, 3b auftreten, an die Zentraleinheit 6 weiterzuleiten. Die Drucksensoren 4a, 4b, 5a, 5b der Zentraleinheit 6 sind so mit den Luftpumpen 9a, 9b, 9c, 9d verbunden, dass sie jeweils den Druck sensorisieren können, der durch die jeweilige Zuführung 7a, 7b, 7c, 7d von den Manschetten 2a, 2b, 3a, 3b übertragen wird.

Die Luftpumpen 9a, 9b, 9c, 9d können Luft gemäß einer von einem Benutzer der Vorrichtung 1 in die Zentraleinheit 6 eingegebenen Auswahl pumpen, also beispielsweise eine große Menge Luft in einer kurzen Zeitdauer oder eine kleine Menge Luft in einer großen Zeitdauer in die Manschetten 2a, 2b, 3a, 3b pumpen.

Die Ventilsteuerung 8 ist in der Lage, in den Manschetten 2a, 2b, 3a, 3b bzw. in der Zentraleinheit 6 angeordnete Ventile (nicht dargestellt) zu steuern, so dass das Auslassen von Luft aus den Manschetten 2a, 2b, 3a, 3b möglich ist. Dabei steuert die Ventilsteuerung 8 die Ventile gemäß einer von einem Benutzer in die Vorrichtung 1 eingegebenen Auswahl über die Menge und Dauer des Luftauslassens oder gemäß einem entsprechenden Steuerprogramm.

Eine in der Zentraleinheit 6 vorgesehene Pumpensteuerung kann ein Aufpumpen der Manschetten 2a, 2b, 3a, 3b regeln, indem während des Aufpumpens der Manschetten 2a, 2b, 3a, 3b der jeweilige Blutdruck der Extremität gemessen wird, an welcher die jeweiligen Manschetten anliegen. Entsprechend der ermittelten Druckwerte regelt die Pumpensteuerung das Aufpumpen jeder der Manschetten 2a, 2b, 3a, 3b auf das jeweils optimale Druckniveau durch die jeweilige Luftpumpe 9a, 9b, 9c, 9d.

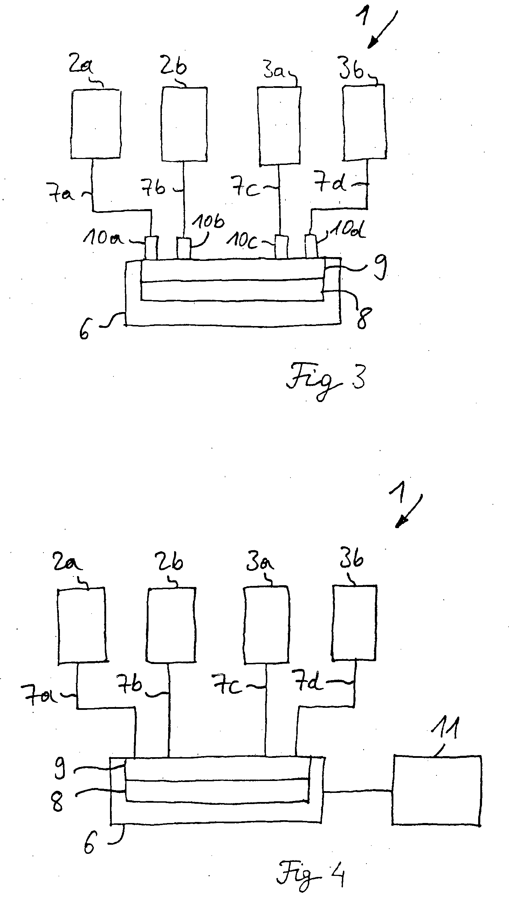

Figur 3 zeigt eine Skizze einer Vorrichtung 1 zum Bestimmen eines Knöchel-Arm-Indexes eines Probanden. Einzelheiten zu Figur 3 werden nur in soweit erläutert, wie sie Unterschiede zu der Ausführungsform nach Figur 2 betreffen.

Die Vorrichtung 1 weist lediglich eine Luftpumpe 9 auf, durch welche sich entsprechend einer Steuerung bzw. Regelung durch eine Pumpensteuerung die Manschetten 2a, 2b, 3a, 3b aufpumpen lassen. Zwischen den Zuführungen 7a, 7b, 7c, 7d und der Luftpumpe 9 sind Rückschlagventile 10a, 10b, 10c, 10d angeordnet, durch welche eine Druckentkopplung der Manschetten 2a, 2b, 3a, 3b und der Luftpumpe 9 besteht. Die Drucksensoren zum Sensorisieren der jeweiligen Drücke an den Manschetten 2a, 2b, 3a, 3b sind nicht dargestellt, sind jedoch zwischen den Manschetten 2a, 2b, 3a, 3b und den jeweiligen Rückschlagventilen 10a, 10b, 10c, 10d angeordnet.

Die Pumpensteuerung steuert die Luftpumpe 9 derart an, dass bereits während der Aufpumpphase der Blutdruck gemessen wird. Aufgrund der Messergebnisse wird der erforderliche Aufpumpdruck in den Manschetten 2a, 2b, 3a, 3b ermittelt, wobei der absolut höchste Aufpumpdruck in einer Manschette dann auch für die anderen Manschetten verwendet wird bzw. für die anderen Manschetten maßgeblich ist.

Die Ventilsteuerung 8 steuert das Öffnen und Schließen der Ventile und kann ebenfalls das Öffnen und Schließen der Rückschlagventile 10a, 10b, 10c, 10d steuern.

Figur 4 zeigt eine Skizze einer Vorrichtung 1 zum Bestimmen eines Knöchel-Arm-Indexes eines Probanden. Einzelheiten zu Figur 4 werden nur in soweit erläutert, wie sie Unterschiede zu der Ausführungsform nach Figur 3 betreffen.

Die Zentraleinheit 6 ist mit einem Anzeigeelement 11 verbunden, welches Daten anzeigen kann, die von der Zentraleinheit 6 ermittelt wurden. Beispielsweise kann das Anzeigeelement Druckwerte für an den Manschetten 2a, 2b, 3a, 3b gemessenen Drücke oder daraus ermittelte Knöchel-Arm-Indizes eines Probanden als Werte oder Kurven grafisch anzeigen. Das Anzeigeelement 11 kann Daten aber auch akkustisch signalisieren, indem beispielsweise Töne über Lautsprecher ausgegeben werden.

Bei den dargestellten Vorrichtungen werden die Manschetten 2a, 2b an jeweils einem Oberarm, die Manschetten 3a, 3b an jeweils einem Knöchel eines Probanden angelegt. Die Manschetten 2a, 2b, 3a, 3b werden mit Luft aufgepumpt, oder ein Druck von außen wird auf sie ausgeübt, so dass der somit durch die Manschetten 2a, 2b, 3a, 3b auf die Extremitäten ausgeübte Druck groß genug ist, um den Blutfluss in den Arterien unterhalb der Manschetten 2a, 2b, 3a, 3b zu behindern.

Dann wird der Druck auf die Arterien dadurch verringert, dass Luft aus den Manschetten 2a, 2b, 3a, 3b ausgelassen wird. Dies geschieht vorzugsweise durch eine entsprechende Regelung der Ventilsteuerung 8, welche Ventile in den Manschetten 2a, 2b, 3a, 3b oder in der Zentraleinheit 6 öffnet. Entsprechend ist es aber auch möglich, einen von außen auf die Manschetten 2a, 2b, 3a, 3b ausgeübten Druck zu reduzieren. Durch den verringerten Druck auf die jeweiligen Arterien wird der Blutstau in den Arterien gelöst und die Drucksensoren 4a, 4b der Zentraleinheit 6 können den Blutdruck in den Armen sensorisieren, die Drucksensoren 5a, 5b der Zentraleinheit 6 den Blutdruck in den Beinen. Dabei ist das Sensorisieren des systolischen, des diastolischen sowie des mittleren Blutdrucks der jeweiligen Extremität möglich und die Zentraleinheit 6 ermittelt aus den entsprechenden Messdaten Druckwerte für die jeweiligen Extremitäten nach einem oszillatorischen Messprinzip.

Für den Betrieb der Vorrichtung werden vorzugsweise alle vier Manschetten 2a, 2b, 3a, 3b an den Extremitäten des Probanden angelegt. Die Sensorisierung der Blutdrücke in den Extremitäten erfolgt durch die zur Vereinfachung in Fig. 4 nicht dargestellten Sensoren 4a, 4b, 5a, 5b der Zentraleinheit 6 gleichzeitig und die Zentraleinheit 6 kann diese Messdaten gleichzeitig verarbeiten. Die Zentraleinheit 6 ermittelt aus den Messdaten der Blutdrücke zugehörige Druckwerte für die Extremitäten. Aus dem Quotienten des Druckwerts eines Knöchels des Probanden und dem Mittelwert der Druckwerte seiner Oberarme wird der ABI-Wert für die Körperhälfte des entsprechenden Knöchels bestimmt, also der ABI-Wert links bzw. der ABI-Wert rechts. Der ABI-Wert links bzw. der ABI-Wert rechts des Probanden wird aus dem Quotienten des Druckwerts dieses linken bzw. des rechten Beines und dem Mittelwert der Druckwerte der Arme bestimmt, wenn der Unterschied der Druckwerte der Arme nicht höher als 10mmHg beträgt. Wenn der Unterschied der Druckwerte der Arme über 10mmHg liegt, wird der ABI-Wert links bzw. der ABI-Wert rechts des Probanden aus dem Quotienten des Druckwerts des linken bzw. des rechten Beines und dem höheren der beiden Druckwerte der Arme bestimmt. Durch das gleichzeitige Sensorisieren der Blutdrücke und das gleichzeitige Verarbeiten der Messdaten können die ABI-Werte links und rechts gleichzeitig ermittelt werden.

Die ermittelten Druckwerte der jeweiligen Extremitäten können über das Anzeigeelement 11 grafisch oder akustisch dargestellt werden. Ebenso ist die grafische oder akustische Anzeige der ermittelten Knöchel-Arm-Indizes über das Anzeigeelement 11 möglich.

## Patentansprüche

1. Vorrichtung (1) zum Bestimmen eines Knöchel-Arm-Indexes eines Probanden, mit
- vier an jeweils einer der Extremitäten, insbesondere der Oberarme und der Knöchel, des Probanden anlegbaren, mit Luft aufpumpbare Manschetten (2a, 2b, 2c, 2d), mit denen jeweils ein Druck auf die entsprechende Extremität ausübbar ist;
- jeweils einem einer Manschette (2a, 2b, 2c, 2d) zugeordneten Ventil zum Regeln des Auslassens von Luft aus der entsprechenden Manschette (2a, 2b, 2c, 2d)
- vier Drucksensoren (4a, 4b, 5a, 5b); und
- einer mit den Drucksensoren (4a, 4b, 5a, 5b) verbundenen Zentraleinheit (6) zum oszillometrischen Messen von Blutdrücken des Probanden, die eine Ventilsteuerung (8) zum separaten Steuern der Ventile aufweist; wobei
- durch die Zentraleinheit (6) Druckwerte der jeweiligen systolischen und/oder diastolischen Blutdrücke des Probanden an den Drucksensoren (4a, 4b, 5a, 5b) der Manschetten (2a, 2b, 2c, 2d) ermittelbar sind; **dadurch gekennzeichnet dass**
- die Druckwerte durch die Zentraleinheit (6) gleichzeitig an den Manschetten (2a, 2b, 2c, 2d) ermittelbar sind, wobei
- die Ventilsteuerung die Ventile derart separat steuert, dass mit einem großen Volumen aufgepumpte Manschetten mit einem stärkeren Luftstrom entleerbar und schwächer aufgepumpte Manschetten mit einem kleineren Luftstrom entleerbar sind, so dass die Drücke in den Manschetten synchronisiert immer auf dem gleichen Druckniveau sind und ein gleichmäßiges Auslassen von Luft aus den Manschetten möglich ist.

2. Vorrichtung (1) gemäß Anspruch 1, **dadurch gekennzeichnet, dass** durch die Zentraleinheit (6) ein Knöchel-Arm-Index des Probanden aus zumindest zwei der Druckwerte bestimmbar ist.

3. Vorrichtung (1) gemäß einem der Ansprüche 1 oder 2, **dadurch gekennzeichnet, dass** ein Knöchel-Arm-Index links bzw. rechts aus den Druckwerten jeweils des linken bzw. des rechten Knöchels und der beiden Oberarme durch die Zentraleinheit (6) bestimmbar ist.

4. Vorrichtung (1) gemäß einem der Ansprüche 2 oder 3, **dadurch gekennzeichnet, dass** durch die Zentraleinheit (6) mindestens zwei Knöchel-Arm-Indizes unterschiedlicher Arme oder Beine gleichzeitig bestimmbar sind.

5. Vorrichtung (1) gemäß einem der vorhergehenden Ansprüche, **gekennzeichnet durch** eine mit der Zentraleinheit (6) verbundene Anzeigeeinheit (11) zum Anzeigen der Druckwerte oder der Knöchel-Arm-Indizes.

6. Vorrichtung (1) gemäß einem der vorhergehenden Ansprüche, **dadurch gekennzeichnet, dass** die Zentraleinheit (6) wenigstens eine mit den Manschetten (2a, 2b, 2c, 2d) über jeweils einen Luftschlauch verbundene Luftpumpe (9a, 9b, 9c, 9d) aufweist, durch welche die Manschetten (2a, 2b, 2c, 2d) aufblasbar sind.

7. Vorrichtung (1) gemäß Anspruch 5 oder 6, **dadurch gekennzeichnet, dass** wenigstens eine der Manschetten (2a, 2b, 2c, 2d) ein Ventil zum Regeln des Auslassens von durch die wenigstens eine Luftpumpe (9a, 9b, 9c, 9d) zugeführter Luft aus wenigstens einer der Manschetten (2a, 2b, 2c, 2d) aufweist.

8. Vorrichtung (1) gemäß Anspruch 5 oder 6, **dadurch gekennzeichnet, dass** die Zentraleinheit (6) wenigstens ein Ventil zum Regeln des Auslassens von durch die wenigstens eine Luftpumpe (9a, 9b, 9c, 9d) zugeführter Luft aus wenigstens einer der Manschetten (2a, 2b, 2c, 2d) aufweist.

9. Verfahren zum Bestimmen eines Knöchel-Arm-Indexes eines Probanden, mit folgenden Schritten:
- Anlegen je einer mit einer Drucksensoren (4a, 4b, 5a, 5b) aufweisenden Zentraleinheit (6) verbundenen Manschette (2a, 2b, 2c, 2d) an den Extremitäten des Probanden; **gekennzeichnet durch**
- Entleeren von mit einem großen Volumen aufgepumpten Manschetten mit einem stärkeren Luftstrom und Entleeren von schwächer aufgepumpten Manschetten mit einem kleineren Luftstrom, bei einem
- Einhalten eines gleichen Druckniveaus in den Manschetten, und einem
- gleichmäßigen Auslassen von Luft aus den Manschetten.
- gleichzeitiges oszillometrisches Messen von Blutdrücken an den jeweiligen Extremitäten zum Ermitteln je eines Druckwertes der Blutdrücke, wobei **durch** die Manschetten (2a, 2b, 2c, 2d) Drücke mit einem einheitlichen Druckniveau auf die entsprechenden Extremitäten ausgeübt werden und die Drücke derart verringert werden, dass das entsprechend verringerte Druckniveau einheitlich bleibt; und
- Bestimmen eines Knöchel-Arm-Indexes aus den Druckwerten.

10. Verfahren gemäß Anspruch 9, **dadurch gekennzeichnet, dass** der Schritt zum Anlegen der Manschetten (2a, 2b, 2c, 2d) an den Extremitäten die folgenden Schritte aufweist:
Anlegen je einer Manschette (2a, 2b, 2c, 2d) an jeweils einem Oberarm des Probanden; und
- Anlegen je einer Manschette (2a, 2b, 2c, 2d) an jeweils einem Knöchel des Probanden.

11. Verfahren gemäß Anspruch 9 oder 10, **dadurch gekennzeichnet, dass** der Schritt zum Bestimmen eines Knöchel-Arm-Indexes den folgenden Schritt aufweist:
- Bestimmen eines Knöchel-Arm-Indexes links oder eines Knöchel-Arm-Indexes rechts durch Errechnen des Quotienten des Druckwerts des entsprechenden linken oder rechten Knöchels und der halben Summe der jeweiligen Druckwerte der Ober-arme.

12. Verfahren gemäß Anspruch 9 oder 10, **dadurch gekennzeichnet, dass** der Schritt zum Bestimmen eines Knöchel-Arm-Indexes den folgenden Schritt aufweist:
- Bestimmen eines Knöchel-Arm-Indexes links oder eines Knöchel-Arm-Indexes rechts durch Errechnen des Quotienten des Druckwerts des linken bzw. Knöchels und dem höheren der beiden Druckwerte der Oberarme.

## Claims

1. Device for determining an ankle-brachial index of a test subject, with
- four cuffs (2a, 2b, 2c, 2d), inflatable with air, each mountable on one of the extremities, in particular the upper arm and the ankle, with which a pressure can be exerted on each corresponding extremity;
- a valve associated with each cuff (2a, 2b, 2c, 2d) for regulating the venting of air from the corresponding cuff (2a, 2b, 2c, 2d)
- four pressure sensors (4a, 4b, 5a, 5b); and
- a central unit (6) connected to the pressure sensors (4a, 4b, 5a, 5b) for oscillometric measurement of blood pressure of the test subject, having a valve control (8) for separate control of the valves: whereby
- pressure values of the respective systolic and/or diastolic blood pressures of the test subject
can be determined by the central unit (6) at the pressure sensors (4a, 4b, 5a, 5b) of the cuffs (2a, 2b, 2c, 2d), **characterised in that**
- the pressure values at the cuffs (2a, 2b, 2c, 2d) are determined simultaneously by the central
unit (6), whereby
- the valve control system controls the valves separately so that cuffs inflated with a large volume
can be emptied with a stronger air flow and cuffs inflated to a lesser degree can be emptied with
a weaker air flow, so that the pressures in the cuffs are always synchronised to the same pressure level and a uniform venting of air from the cuffs is possible.

2. Device (1) in accordance with claim 1, **characterised in that** an ankle-brachial index of the test subject can be determined by the central unit (6) from at least two of the pressure values.

3. Device (1) in accordance with one of the claims 1 or 2, **characterised in that** an ankle-brachial index can be determined on the left or on the right by the central unit (6) from the pressure values of respectively the left or right ankle and the two upper arms.

4. Device (1) in accordance with one of the claims 2 or 3, **characterised in that** at least two ankle-brachial indices can be determined simultaneously by the central unit (8) for different arms or legs.

5. Device (1) in accordance with one of the preceding claims, **characterised by** a display unit (11) connected to the central unit (6) for displaying the pressure values or the ankle-brachial index.

6. Device (1) in accordance with one of the preceding claims, **characterised in that** the central unit (6) has at least one air pump (9a, 9b, 9c, 9d), connected by a respective air tube with the cuffs (2a, 2b. 2c, 2d), by means of which the cuffs (2a, 2b. 2c, 2d) can be inflated.

7. Device (1) in accordance with claim 5 or 6, **characterised in that** at least one of the cuffs (2a, 2b. 2c, 2d) has a valve for controlling the venting of air supplied by the at least one air pump (9a, 9b, 9c, 9d) from at least one of the cuffs (2a, 2b. 2c, 2d).

8. Device (1) in accordance with claim 5 or 6, **characterised in that** the central unit (6) has at least one valve for controlling the venting of air supplied by the at least one air pump (9a, 9b, 9c, 9d) from at least one of the cuffs (2a, 2b. 2c, 2d).

9. Procedure for determining an ankle-brachial index of a test subject, comprising the following steps:
- applying each cuff (2a, 2b, 2c, 2d) connected to a central unit (6) having a pressure sensors (4a, 4b, 5a, 5b) to the extremities of the test subject;
- evacuating cuffs inflated with a large volume with a stronger air flow and evacuating cuffs inflated more weakly with a smaller air flow, with
- maintenance an equal pressure level in the cuffs, and
- uniform venting of air from the cuffs,
- simultaneous oscillometric measurement of blood pressures at the respective extremities to determine the pressure value of the blood pressures at each, whereby pressures are exerted at a consistent pressure level by the cuffs (2a, 2b, 2c, 2d) on the corresponding extremities and the pressures are reduced so that the corresponding pressure level remains consistent; and
- determining an ankle-brachial index from the pressure values.

10. Procedure in accordance with claim 9, **characterised in that** the step for applying the cuffs (2a, 2b. 2c, 2d) to the extremities comprises the following steps:
- applying a cuff (2a, 2b. 2c, 2d) to each upper arm of the test subject: and
- applying a cuff (2a, 2b. 2c, 2d) to each ankle of the test subject.

11. Procedure in accordance with claim 9 or 10, **characterised in that** the step for determining an ankle-brachial index comprises the following steps:
- determining an ankle-brachial index on the left or an ankle-brachial index on the right by calculating the ratio of pressure value of the left or right ankle and half the total of the respective pressure values of the upper arms.

12. Procedure in accordance with claim 9 or 10, **characterised in that** the step for determined an ankle-brachial index comprises the following step:
- determining an ankle-brachial index on the left or an ankle-brachial index on the right by calculating the ratio of the pressure value of the left or [*right?*] ankle and the higher of the two pressure values of the upper arms.

## Revendications

1. Dispositif (1) pour déterminer un index cheville-bras d'un sujet, avec
- quatre manchons (2a, 2b, 2c, 2d) qui sont aptes à être appliqués sur les extrémités respectives, en particulier sur les bras et les chevilles, du sujet et qui sont aptes à être gonflés avec de l'air, et avec lesquels une pression est apte à être exercée sur l'extrémité correspondante ;
- une soupape associée à chacun des manchons (2a, 2b, 2c, 2d) pour réguler l'évacuation de l'air hors du manchon correspondant (2a, 2b, 2c, 2d) ;
- quatre capteurs de pression (4a, 4b, 5a, 5b) ; et
- une unité centrale (6), reliée aux capteurs de pression (4a, 4b, 5a, 5b), pour la mesure oscillométrique des pressions artérielles du sujet, qui présente une commande de soupapes (8) pour une commande séparée des soupapes ; étant précisé
- que grâce à l'unité centrale (6), des valeurs des pressions artérielles systolique et/ou diastolique du sujet peuvent être déterminées au niveau des capteurs de pression (4a, 4b, 5a, 5b) des manchons (2a, 2b, 2c, 2d) ; **caractérisé en ce que**
- les valeurs de pression sont aptes à être déterminées par l'unité centrale (6) simultanément au niveau des manchons (2a, 2b, 2c, 2d), étant précisé
- que la commande de soupapes commande les soupapes séparément de telle sorte que les manchons gonflés avec un grand volume puissent être vidés avec un courant d'air plus fort et que les manchons gonflés plus faiblement puissent être vidés avec un plus petit courant d'air, de sorte que les pressions dans les manchons sont toujours au même niveau, de manière synchronisée, et qu'une évacuation régulière de l'air hors des manchons est possible.

2. Dispositif (1) selon la revendication 1, **caractérisé en ce que** grâce à l'unité centrale (6), un index cheville-bras du sujet peut être déterminé à partir d'au moins deux des valeurs de pression.

3. Dispositif (1) selon l'une des revendications 1 ou 2, **caractérisé en ce qu'**un index cheville-bras est apte à être déterminé par l'unité centrale (6) à gauche ou à droite à partir des valeurs de pression de la cheville gauche ou droite, respectivement, et des deux bras.

4. Dispositif (1) selon l'une des revendications 2 ou 3, **caractérisé en ce que** grâce à l'unité centrale (6), au moins deux index cheville-bras de bras ou de jambes différents sont aptes à être déterminés simultanément.

5. Dispositif (1) selon l'une des revendications précédentes, **caractérisé par** une unité d'affichage (11), reliée à l'unité centrale (6), pour afficher les valeurs de pression ou les index cheville-bras.

6. Dispositif (1) selon l'une des revendications précédentes, **caractérisé en ce que** l'unité centrale (6) comporte au moins une pompe à air (9a, 9b, 9c, 9d) qui est reliée aux manchons (2a, 2b, 2c, 2d) par des tuyaux à air respectifs et grâce à laquelle les manchons (2a, 2b, 2c, 2d) peuvent être gonflés.

7. Dispositif (1) selon la revendication 5 ou 6, **caractérisé en ce que** l'un au moins des manchons (2a, 2b, 2c, 2d) comporte une soupape pour réguler l'évacuation, hors de l'un au moins des manchons (2a, 2b, 2c, 2d), de l'air amené par la ou les pompes à air (9a, 9b, 9c, 9d).

8. Dispositif (1) selon la revendication 5 ou 6, **caractérisé en ce que** l'unité centrale (6) comporte au moins une soupape pour réguler l'évacuation, hors de l'un au moins des manchons (2a, 2b, 2c, 2d), de l'air amené par la ou les pompes à air (9a, 9b, 9c, 9d)

9. Procédé pour déterminer un index cheville-bras d'un sujet, avec les étapes qui consistent :
- à appliquer sur chacune des extrémités du sujet un manchon (2a, 2b, 2c, 2d) relié à une unité centrale (6) qui comporte des capteurs de pression (4a, 4b, 5a, 5b) ;
**caractérisé par** les étapes qui consistent
- à vider avec un courant d'air plus fort les manchons gonflés avec un grand volume, et à vider avec un courant d'air plus petit les manchons plus faiblement gonflés,
- en respectant un même niveau de pression dans les manchons, et
- en évacuant de manière régulière l'air des manchons,
- à procéder en même temps à une mesure oscillométrique des pressions artérielles à chacune des extrémités, étant précisé que grâce aux manchons (2a, 2b, 2c, 2d), des pressions de niveau uniforme sont exercées sur les extrémités correspondantes, et que les pressions sont réduites de telle sorte que le niveau de pression réduit de manière appropriée reste uniforme ; et
- à déterminer un index cheville-bras à partir des valeurs de pression.

10. Procédé selon la revendication 9, **caractérisé en ce que** l'étape pour appliquer le manchon (2a, 2b, 2c, 2d) sur les extrémités comporte les étapes qui consistent :
- à appliquer un manchon (2a, 2b, 2c, 2d) sur chaque bras du sujet ; et
- à appliquer un manchon (2a, 2b, 2c, 2d) sur chaque cheville du sujet.

11. Procédé selon la revendication 9 ou 10, **caractérisé en ce que** l'étape pour déterminer un index cheville-bras comporte l'étape qui consiste :
- à déterminer un index cheville-bras à gauche, ou un index cheville-bras à droite grâce au calcul du quotient de la valeur de pression de la cheville gauche ou droite correspondante et de la demi-somme des valeurs de pression respectives des bras.

12. Procédé selon la revendication 9 ou 10, **caractérisé en ce que** l'étape pour déterminer un index cheville-bras comporte l'étape qui consiste :
- à déterminer un index cheville-bras à gauche ou un index cheville-bras à droite grâce au calcul du quotient de la valeur de pression de la cheville gauche ou droite correspondante et de la plus élevée des deux valeurs de pression des bras.
